# EUROPEAN PATENT APPLICATION

(11) **EP 2 562 243 A1**
(43) Date of publication of application: **27.02.2013**
(21) Application number: 12178111.6
(22) Date of filing: 26.07.2012
(51) Int. Cl.: C12M 1/00

(54) **Operation of fermentation devices**

(30) Priority: 26.08.2011 EP 11179096
(71) Applicant: Thöni Industriebetriebe GmbH, 6410 Telfs (AT)
(72) Inventor: Krismer, Michael, 6500 Landeck (AT)
(74) Representative: Dilg, Haeusler, Schindelmann Patentanwaltsgesellschaft mbH

(57) **Abstract**

In accordance with embodiments there is provided a method of operating a fermentation device (100) comprising a fermenter (102), the method comprising: moving an organic material through a first zone (106) of the fermenter (102) in an downstream direction (104) while allowing fermentation of the organic material; removing a first part (C) of the organic material from the first zone (106) at a first location (113); moving a remaining second part (F2) of the organic material in the downstream direction (104) past the first location (113) into a second zone (108) of the fermenter (102); re-introducing the first part (C) of the organic material into the fermenter (102) at a second location (115) upstream the first location (113).

## Description

### FIELD OF THE INVENTION

The present invention relates to the field of fermentation devices.

### BACKGROUND OF THE INVENTION

In fermentation devices it is known to use material out of the fermenter for inocculation of fresh material introduced in the fermenter.

EP 1 095 924 A2 discloses to use only press water obtained from dewatering of a fermented material for inoculation of a fresh material introduced in the fermenter.

In view of the above described situation, there exists a need for a method of operating a fermentation device with improved characteristics.

### SUMMARY OF THE INVENTION

This need may be met by the subject matter according to the independent claims. Advantageous embodiments of the herein disclosed subject matter are described by the dependent claims.

According to an embodiment of a first aspect of the herein disclosed subject matter there is provided a method of operating a fermentation device comprising a fermenter, the method comprising: moving an organic material through a first zone of the fermenter in a downstream direction while allowing fermentation of the organic material; removing a first part of the organic material from the first zone at the first location; moving a remaining second part of the organic material in the downstream direction past the first location into a second zone of the fermenter; reintroducing the first part of the organic material into the fermenter at a second location upstream the first location.

By varying an amount of reintroduced material per time unit, a circulation rate of organic material that is circulated through the first zone is varied. Generally, by changing the circulation rate of the organic material through the first zone, the fermentation conditions in the fermenter may be optimized. Further, the establishment of two different zones in the fermenter allows an adaption of a moving speed of the material in the first zone while maintaining an overall throughput through the fermenter.

According to an embodiment, the removal of the first part of the organic material is performed continuously. According to another embodiment the removal of the first part of organic material is performed discontinuously, e.g. intermittently.

According to an embodiment, the moving speed of the organic material through the first zone is determined by the input rate of organic material into the first zone. In accordance with an embodiment, a rate as recited herein is defined as mass per time unit. In a so-called plug-flow fermenter the fermentation material is moved through the fermenter by introducing fresh material at a fermenter input of the fermenter and discharging fermented material at a fermenter output of the fermenter. In accordance with embodiments of the herein disclosed subject matter, the "plug flow" is modified by providing two different zones in the fermenter. In accordance with an embodiment, in a single fermenter two plugs with different moving speeds are provided.

According to a further embodiment, a moving device may be provided for moving in the organic material through the first zone of the fermenter or for assisting in the movement of the organic material through the first zone of the fermenter. For example, in an embodiment a stirring device located in the fermenter may be adapted for assisting or promoting the moving of the organic material through the first zone of the fermenter.

According to a further embodiment, removing a first part of the organic material from the first zone is performed by providing an intermediate outlet at the first location, thereby allowing organic material to be discharged at the first location. Such a discharge of organic material at the first location may occur due to a pressure difference between the organic material in the fermenter and the surrounding. For example, if the intermediate outlet at the first zone is located in the lower part of the fermenter, the hydrostatic pressure which is present in the organic material will force the first part of the organic material out of the fermenter through the intermediate outlet. In order to change the amount of the first part of the organic material per time unit, the intermediate outlet may be closed or reduced in its diameter. According to other embodiments, a conveyor device may be provided for conveying away material removed from the first zone from the intermediate outlet. According to an embodiment, the amount of removed material per time unit may be adjusted by adjusting the conveying speed of the conveyor device.

According to an embodiment, moving a remaining second part of the organic material in the downstream direction past the first location into a second zone of the fermenter is a passive movement occurring due to the introduction of fresh material at the fermenter input and removing at the first zone only part of the organic material in the first zone. Therefore, according to the overall movement of organic material in the downstream direction, the remaining second part of organic material which is not removed at the first location, automatically moves into the second zone of the fermenter. According to another embodiment, a conveyor device may be provided in the fermenter for assisting or promoting movement of the remaining second part of the organic material in the downstream direction past the first location into the second zone of the fermenter.

Reintroducing the first part of the organic material into the fermenter at the second location may be performed passively without conveyor device for actively conveying the first part of organic material into the fermenter at the second location. According to another embodiment, a conveyor device is provided for introducing the first part of the organic material into the fermenter at the second location. For example, according to an embodiment a conveyor device is provided, the conveyor device being adapted for removing the first part of organic material from the first zone at the first location and reintroducing the first part of organic material into the fermenter at the second location. For example in an embodiment such a conveyor device maybe comprises a screw conveyor.

According to an embodiment, the first zone and the second zone of the fermenter are operational zones which are determined by removing the first part of organic material at the first location and reintroducing the first part of organic material at the second location. In a further embodiment, the first zone extends between the first location and the second location. According to a still further embodiment, the first zone and the second zone are defined only by the different flow characteristics (e.g. moving speeds of organic material) in the zones during operation of the fermenter. According to an embodiment, the cross-sectional area of the fermenter in the first zone is equal to the cross-sectional area of the fermenter in the second zone. For example, in an embodiment, the fermenter wall in the first zone, the fermenter wall in the second zone and the fermenter wall in an intermediate zone between the first zone and the second zone are continuous and aligned with respect to each other. For example, in an embodiment, a fermenter in accordance with embodiments of the herein disclosed subject matter may be provided by providing an existing plug flow fermenter with an intermediate outlet and a transfer path in accordance with embodiments of the herein disclosed subject matter. According to another embodiment, the cross-sectional area of the fermenter in the first zone is different from the cross-sectional area in the second zone.

In accordance with an embodiment, the moving speed of the organic material in the first zone is higher than the moving speed of the organic material in the second zone. For example, in an embodiment where the cross-sectional area of the first zone is equal to the cross-sectional area of the second zone, the moving speed of the organic material in the first zone is higher than the moving speed of the organic material in the second zone due to the additional recirculation of the first part of organic material in the first zone.

According to a further embodiment, the method comprises adding a further material to the first part of organic material before introduction of the first part of organic material into the fermenter; and introducing both materials together in the fermenter at the second location. According to an embodiment, the further material is a liquid. According to a further embodiment, the further material is organic material, e.g. fresh organic material. In such a case, both organic materials are introduced into the fermenter at the second location. Introducing both organic materials together into the fermenter into the second location has the advantage that only one input is necessary for introducing both, the fresh organic material and the first part of organic material removed at the first location.

According to an embodiment, the further material may be mixed with the first part of organic material before introduction at the second location, thereby providing a mixture of both materials. According to other embodiments, the further material may be added to the first part of organic material without mixing the both materials. In still other embodiments, the further material and the first part of organic material may be introduced into the fermenter at subsequent, different time intervals.

While in an embodiment the further material is fresh organic material as mentioned above, according to a further embodiment, the fresh organic material and the first part of organic materials are introduced into the fermenter through different inlets.

According to a further embodiment, the method comprises, before introducing both materials into the fermenter, adjusting a dry substance content of the mixture of both materials. By adjusting a dry substance content of the organic material introduced at the second location of the fermenter, an adjustment of the dry substance content within the fermenter can be reduced or even be eliminated. According to an embodiment, where the further material is a liquid, adjusting the dry substance content of both materials is performed by adding a respective amount of the liquid to the first part of organic material.

According to an embodiment, the method comprises adding fresh water to the first part of organic material or, in another embodiment, to the mixture of fresh organic material and the first part of organic material in order to adjust the dry substance content of the resulting mixture. According to a further embodiment, the method comprises adding press water to the first part of organic material to thereby adjust the dry substance content of the resulting mixture (this is also referred to as adjusting the dry substance content of the first part of organic material). It is noted that press water in the sense of the herein disclosed subject matter includes any liquid material obtained from dewatering the output material of the fermenter. Therefore, usually the press water includes a certain content of organic material that depends on the type and amount of fresh organic material that is introduced into the fermenter, as well as on the fermentation conditions in the fermenter and in particular to the fermentation conditions in the first zone and in the second zone.

In accordance with a further embodiment, the method comprises blocking movement of the organic material in a surface region of the organic material at a third location. It was found that surface streams may occur in the fermentation material in the fermenter wherein the moving speed of the organic material in the surface flow is much higher than the average moving speed of the organic material through the fermenter. Therefore, surface flows may reduce the average retention time of the organic material in the fermenter and may lead to an incomplete fermentation therefore to an inefficient operation of the fermenter. By blocking the movement of organic material in the surface region, such surface streams can be blocked or at least reduced and therefore may improve the efficiency of the fermentation device. According to an embodiment, the third location is arranged in the first zone. According to a further embodiment, the third location is arranged in the second zone. According to another embodiment, a plurality of third locations is provided where the movement of organic material in a surface region of the organic material is blocked. Blocking of the movement of the organic material in a surface region of the organic material may be performed e.g. by providing at least one retaining element in the surface region of the third location. According to an embodiment, a retaining element is a plate that extends into the organic material of the fermenter from above.

According to an embodiment of a second aspect of the herein disclosed subject matter, a fermentation device is provided, the fermentation device comprising: a fermenter for fermentation of an organic material during a movement of the organic material through the fermenter in a downstream direction, the fermenter comprising: a first zone with an intermediate outlet for removal of first part of the organic material from the fermenter; a second zone for receiving a remaining second part of the organic material; and an inlet upstream the intermediate outlet; wherein the fermentation device further comprises a transfer path between the intermediate outlet and the inlet, the transfer path being configured for reintroducing the first part of the organic material into the fermenter through the inlet.

According to an embodiment, the intermediate outlet is provided at a first location as described with embodiments of the herein disclosed subject matter. According to a further embodiment, the inlet is provided at a second location as described with embodiments of the herein disclosed subject matter.

According to an embodiment, the fermenter is similar to a plug-flow fermenter where the organic material is moved through the fermenter in a horizontal direction, such that the downstream direction is a horizontal direction. According to an embodiment, a fermenter in accordance with embodiments of the herein disclosed subject matter differs from a plug flow fermenter by the intermediate outlet and the transfer path as disclosed herein. According to an embodiment, a stirring device is provided in the fermenter for stirring the content of the fermenter, thereby mixing vertical portions of fermenter material, such as a floating layer or a sediment layer with the remaining content of the fermenter.

According to an embodiment, the inlet which is coupled to the transfer path is also used for introduction of a further material into the fermenter. For example, in an embodiment, the further material is fresh organic material. In another embodiment, the further material is a liquid. According to another embodiment, a separate inlet for introduction of fresh organic material into the fermenter is provided. According to an embodiment, the fermenter comprises a further outlet for outputting fermented output material of the fermenter which is subsequently provided to a dewatering device for separation of the fermented output of the fermenter into press water and remaining, dewatered organic material. According to an embodiment, the remaining organic material may be subjected to aerobic rotting, resulting e.g. in compost.

According to an embodiment, the transfer path is provided by or contains any suitable means, e.g. tubes, pipes and/or the like. According to an embodiment, the transfer path is configured for preventing an introduction of oxygen into the transfer path. This is in particular useful if the fermenter is operated for anaerobic fermentation of organic material. According to an embodiment, the fermentation device further comprises a feeding path running into the transfer path for adding the further material to the first part of organic material. According to an embodiment, the crossing of the feeding path and the transfer path is located in the vicinity of the inlet. According to another embodiment, the crossing of the feeding path and the transfer path is located in the vicinity of the intermediate outlet of the fermenter.

According of an embodiment, the transfer path further comprises a mixing unit for mixing the further material and the first part of organic material, thereby providing a mixture of both materials. According to an embodiment, the mixing unit is located downstream the crossing between the feeding path and the transfer path. According to a further embodiment, the crossing of the feeding path and the transfer path is provided by the mixing unit. The mixing unit may be provided in any suitable form, e.g. in the form of a mixing path or in the form of a mixing device, e.g. a screw conveyor.

According to an embodiment, the transfer path comprises a conveyor device for conveying the first part of the organic material to the inlet at the second location of the fermenter. If a feeding path is provided, the conveyor device may be provided in the transfer path upstream the crossing between the transfer path and the feeding path. According to a further embodiment, the conveyor device in the transfer path is provided downstream the crossing between the feeding path and the transfer path. According to a further embodiment, the crossing between the feeding path and the transfer path is provided by the conveyor device. According to an embodiment, the conveyor device is configured for mixing the further material and the first part of organic material, thereby providing the mixture of both materials. For example, in an embodiment, the conveyor device is a screw conveyor.

According to an embodiment, the fermentation device is adapted such that the moving speed of the organic material in the first zone is higher than the moving speed of the organic material in the second zone. If the first zone and the second zone have the same or a similar cross-section, the different moving speed may be obtained by the circulation of the first part of the organic material through the first zone. In this regard it is noted that according to an embodiment the removal of the first part of the organic material and its reintroduction through the inlet into the fermenter is continuously performed or at least be performed in a certain time interval. Hence the expression "first part" does not mean that this first part is a specific portion of the organic material but rather that the organic material arriving at the first location is divided into a part (first part) that is transferred to the inlet and reintroduced into the fermenter and a remaining part (second part) which is further moved through the fermenter into the second zone.

According to a further embodiment, the moving speed of the organic material during its movement through the fermenter is influenced by a change of the cross-section of the fermenter in downstream direction. Generally, by changing the moving speed of the organic material during its movement through the fermenter, the fermentation conditions in the fermenter may be optimized.

According to an embodiment, the fermentation device comprises a liquid inlet for adding liquid to the first part of organic material to thereby adjust the dry substance content of the first part of organic material.

According to a further embodiment, the fermentation device comprises a liquid inlet for adding liquid to the mixture of the fresh organic material and the first part of organic material to thereby adjust the dry substance content of the mixture of both organic materials.

According to an embodiment, the liquid inlet is located ahead of the inlet of the fermenter at the second location. According to further embodiment, the liquid inlet is provided at or in the transfer path. According to a further embodiment, the liquid inlet is provided in the feeding path. According to a further embodiment, liquid inlet is provided by the feeding path and the feeding path is configured for adding liquid to the transfer path. According to a further embodiment, the liquid inlet is provided in the mixing unit or in the conveyor device of the transfer path.

According to an embodiment, the liquid comprises of press water. According to a further embodiment, the liquid comprises fresh water. For example, in accordance with both latter embodiments, the liquid may comprise press water and/or fresh water. In accordance with common terminology, press water is liquid that is obtained from dewatering a fermenter output material.

In accordance with a further embodiment, the fermentation device further comprises a conveyor device for conveying the first part of organic material to the inlet.

According to a further embodiment, the fermentation device further comprises a retaining element blocking movement of the organic material in the fermenter in a surface region of the organic material at a third location.

According to an embodiment, a single retaining element is provided in the fermenter. According to another embodiment, two or more retaining elements are provided in the fermenter, the at least two retaining elements being spaced in the downstream direction.

According to embodiments of the second aspect, the fermentation device is adapted for providing the functionality of one or more of the aforementioned embodiments, e.g. the embodiments of the first aspect and/or for providing the functionality as required by one or more of the afore mentioned embodiments, e.g. of the embodiments of the first aspect.

According to an embodiment of a third aspect, a control unit for a fermentation device is provided. The control unit being configured for carrying out the method according to one or more of the embodiments of the first aspect.

According to an embodiment, the control unit is adapted for providing the functionality of one or more of the aforementioned embodiments and/or for providing the functionality as required by one or more by the aforementioned embodiments, e.g. of the embodiments of the first aspect and the second aspect.

According to an embodiment of a fourth aspect of the herein disclosed subject matter, a computer program is provided, the computer program being adapted for, when being executed by a data processor device, controlling the method as set forth in one or more embodiments of the first aspect.

According to embodiments of the fourth aspect, the computer program is adapted for providing the functionality of one or more of the aforementioned embodiments and/or for providing the functionality as required by one or more of the aforementioned embodiments.

As used herein, reference to a computer program is intended to be equivalent to reference to a program element and/or to a computer readable medium containing instructions for controlling a computer system to effect and/or coordinate the performance of the above described method.

The computer program may be implemented as computer readable instruction code by use of any suitable programming language, such as, for example, JAVA, C++, and may be stored on a computer-readable medium (removable disk, volatile or non-volatile memory, embedded memory/processor, etc.). The instruction code is operable to program a computer or any other programmable device to carry out the intended functions. The computer program may be available from a network, such as the World Wide Web, from which it may be downloaded.

According to an embodiment, the invention is realized by means of a computer program respectively software. However, the invention may also be realized by means of one or more specific electronic circuits respectively hardware. Furthermore, the invention may also be realized in a hybrid form, i.e. in a combination of software modules and hardware modules.

In the above there has been described in the following there will be described exemplary embodiments of the subject matter disclosed herein with reference to a fermentation device, a method of operating a fermentation device, a control unit and a computer program. It has to be pointed out that of course any combination of features relating to different aspects of the herein disclosed subject matter is also possible. In particular, some embodiments have been or will be described with reference to apparatus type embodiments, whereas other embodiments have been or will be described with reference to method type embodiments. However, a person skilled in the art will gather from the above and from the following description that, unless otherwise notified, in addition to any combination of features belonging to one aspect also any combination between features relating to different aspects or embodiments, for example even between features of the apparatus type embodiments and features of the method type embodiments is considered to be disclosed with this application.

The aspects and embodiments defined above and further aspects and embodiments of the herein disclosed subject matter are apparent from the examples to be described herein after and are explained with reference to the drawings but to which the invention is not limited.

### BRIEF DESCRIPTION OF THE DRAWINGS

Fig. 1 shows a top view of a fermentation device in accordance with embodiments of the herein disclosed subject matter.
Fig. 2 shows a side view of the fermenter of the fermentation device in Fig. 1 when viewed from line II-II in Fig. 1.
Fig. 3 shows a lower part of the fermenter of Fig. 2 in cross-sectional view along line III-III of Fig. 2.

### DETAILED DESCRIPTION

The illustration in the drawings is schematic. It is noted that in different figures, similar or identical elements are provided with the same reference signs or with reference signs, which are different from the corresponding reference signs only within the first digit.

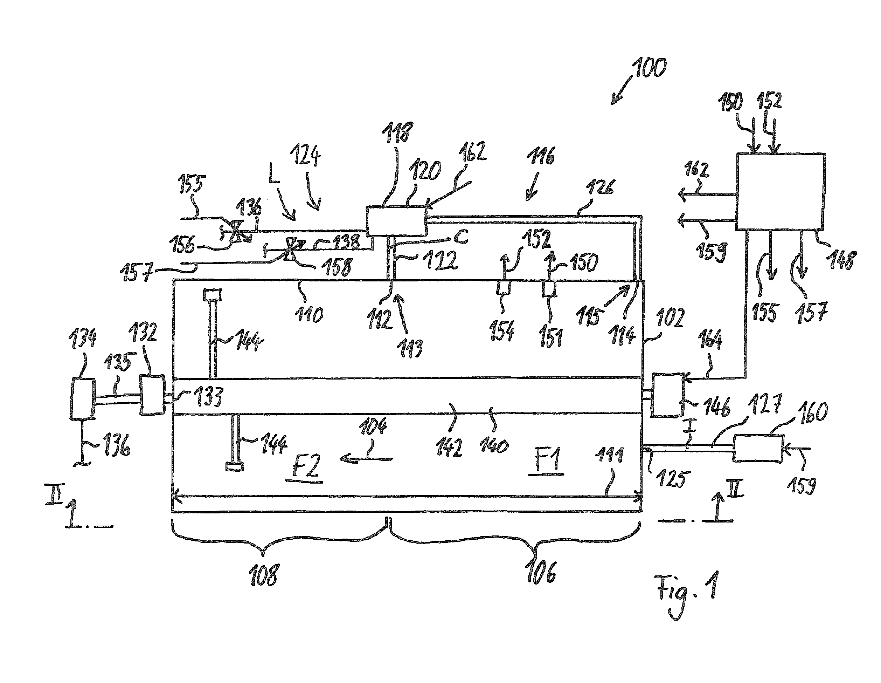

Fig. 1 shows a top view of a fermentation device 100 in accordance with embodiments of the herein disclosed subject matter.

The fermentation device 100 comprises a fermenter 102 for fermentation of organic material during a movement of the organic material through the fermenter 102 in a downstream direction 104. The fermenter comprises a first zone 106 and a subsequent second zone 108. In accordance with an embodiment, the first zone 106 and the second zone 108 are not distinguished by any structural feature on a fermenter wall 110, although such features may be present in other embodiments.

The first zone 106 comprises an intermediate outlet 112 for removal of a first part of the organic material (not shown in Fig. 1) from the fermenter 102 at a first location 113. The second zone 108 receives the remaining second part of organic material that is not removed through the intermediate outlet. The fermenter 102 further comprises an inlet 114 which is located upstream the intermediate outlet 112 at a second location 115.

According to an embodiment, the first zone 106 has a length in range between 20 % and 80 % of the length 111 of the fermenter cavity. According to a further embodiment, the first zone 106 has a length in range between 30 % to 70 % of the length 111 of the fermenter cavity. For example, in an embodiment, the intermediate outlet 112 is located in the middle of the fermenter cavity, corresponding to a length of the first zone 112 that is equal to 50 % of the length 111 of the fermenter cavity. According to an embodiment, the length 111 of the fermenter cavity as well as the length of the first zone and the second zone is measured along the downstream direction 104. For example, in accordance with an exemplary embodiment shown in Fig. 1, the length of the fermenter cavity is the length of the first zone plus the length of the second zone. According to an embodiment, the length of the first zone 106 is the distance between the inlet 114 and the intermediate outlet 112.

The fermentation device further comprises a transfer path 116 extending between the intermediate outlet 112 and the inlet 114. The transfer part 116 is configured for reintroducing the first part of organic material that is removed from the fermenter through the intermediate outlet 112, again into the fermenter 102 through the inlet 114.

In accordance with a further embodiment, the transfer path 116 comprises a mixing unit 118 and a conveyor device 120 in combined form, e.g. in the form of a screw conveyor as shown in Fig. 1. The screw conveyor 118, 120 receives the first part of organic material from the intermediate outlet 112 through a duct 122 which extends between the intermediate outlet 112 and the mixing unit 118 and/or the conveyor device 120. The screw conveyor 118, 120 further receives a further material through a feeding path 124. In the screw conveyor 118, 120 the two input materials, i.e. the further material and the first part of organic material are mixed and a respective mixture of both materials is provided into an output duct 126 which extends between the screw conveyor 118, 120 and the inlet 114. During operation, the screw conveyor transfers the mixture of both materials through the output duct 126 and through the inlet 114 into the fermenter 102.

The removal of the first part of the organic material through the intermediate outlet 112 generates the first material flow rate F1 in the first zone and the second material flow rate F2 in the second zone. It is noted that as usual, herein the material flow rate is defined by mass of material per second. In other words, the material flow rate is defined by a certain amount of material flowing through a cross-section of the fermenter per time unit. Since the first part of organic material is removed at the intermediate outlet 112 and reintroduced to the inlet 114, between the inlet 114 and the intermediate outlet 112 the flow rate F1 is the flow of the remaining material F2 plus an additional flow rate of circulated material C that is recirculated through the transfer path 116. Hence besides some material loss, e.g. through the generation of gaseous fermentation products, the material flow rate F2 corresponds approximately to input material flow rate I which is the flow rate of fresh material introduced into the fermenter and the flow rate L which is the flow rate of liquid introduced into the fermenter, e.g. for adjustment of a dry substance content. In accordance with an embodiment, for introduction of fresh organic material into the fermenter a further inlet 125 and a further feeding path 127 is provided. The further feeding path 127 is configured for introducing the fresh organic material into the fermenter at the input material flow rate I. In accordance with an embodiment, a conveyor device 160 is provided for conveying the fresh organic material along the further feeding path 127 through the further inlet 125 into the fermenter 102.

Hence, in accordance with an embodiment, the material flow rate F1 in the first zone 106 is higher than the material flow rate F2 in the second zone 108. The different material flow rates in the first zone 106 and in the second zone 108 allow for a more precise adjustment of the fermentation processes of the fermentation material on its way through the fermenter 102. A further advantage of the removal of the first part of organic material at the intermediate outlet 112 is the fact that the length of the transfer path 116 is shorter than a length of an inoculation line that is conventionally provided from a dewatering device 134 to the inlet 114 of the fermenter.

In accordance with an embodiment, the fermenter 102 comprises a further outlet 133, also referred to as fermenter outlet, through which the fermentation material from the second zone is discharged. From the fermenter outlet 133 a fermenter outlet duct 135 is provided to the dewatering device 134. In accordance with an embodiment, the dewatering device 134 separates the fermented material received from the fermenter outlet 133 into a dewatered material portion and press water. The press water is provided at a press water line 136. In accordance with an embodiment, the press water line 136 extends to the mixing unit 118, thereby allowing to adjust the dry substance content of the first part of organic material fed to the fermenter via the inlet 114. In accordance with an embodiment, a fresh water supply line 138 is provided alternatively to the press water line 136 or additionally to the press water line 136, as shown in Fig. 1. The fresh water line 138 allows the addition of fresh water to the first part of organic material to thereby adjust the dry substance content of the mixture of both organic materials without adding too much press water to the mixture. In accordance with an embodiment, the press water line 136 and the fresh water supply line 138 each form a feeding path 124 according to embodiments of the herein disclosed subject matter. Further, the sum of the supply rates of fresh water and press water corresponds to the liquid flow rate L.

In accordance with an embodiment, the fermenter comprises a stirring device 140 which according to an embodiment comprises a rotatable shaft 142 to which stirring paddles 144 are mounted, two of which are shown in Fig. 1. The rotatable shaft is driven by a motor 146 to thereby provide for a mixing of the content of the fermenter 102. In particular, the stirring device 140 provides for a mixing of a floating layer of organic material together with a remaining portion of the organic material. Further, the stirring device 140 may be adapted for loosening a sediment layer at the bottom of the fermenter 102, thereby reducing the sediment layer.

In accordance with a further embodiment, a control unit 148 for the fermentation device is provided, the control unit being configured for controlling operation of the fermentation device, for example by contolling one or more of the entities of the fermentation device, e.g. the mixing unit 118, the conveyor device 120, the motor 146 of the stirring device 140, the dewatering device 134, the discharge pump 132, etc. According to an embodiment, the control unit 148 receives sensor signals, for example a level signal 150 of a level sensor 151 sensing the filling level of the fermenter with organic material. According to a further embodiment, the control unit 148 receives a temperature signal 152 from one or more temperature sensors 154.

Depending on a predefined control scheme and/or received sensor signals 150, 154, the control unit provides control signals to entities of the fermentation device, e.g. one or more of
i) a control signal 155 to valve 156 and/or a control signal 157 to valve 158 for controlling the feeding of liquid to the mixing device 118,
ii) a control signal 159 to the conveyor device 160 for controlling an amount or a material rate I of fresh material to be introduced into the fermenter 102,
iii) a control signal 162 to the conveyor device 120 for adjusting a conveying speed and hence the material flow rate introduced into the fermenter 102 through the inlet 114, and
iv) a control signal 164 for controlling the operation of the stirring device 140, for example, in an embodiment, to the motor 146.

According to other embodiments, further control signals are provided by the control unit 148 to entities of the fermentation device.

According to an embodiment, the intermediate outlet 112 comprises a single outlet opening. According to another embodiment, the intermediate outlet 112 comprises two or more outlet openings which may, in a respective embodiment, be spaced in a circumferential direction along the wall of the fermenter 102.

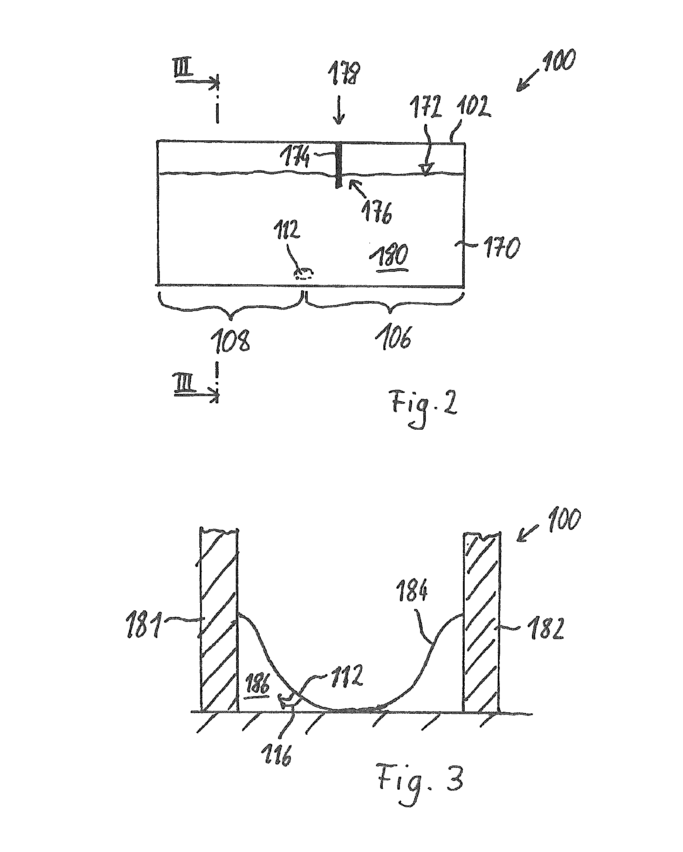

Fig. 2 shows a side view of the fermenter 102 of the fermentation device 100 in Fig. 1 when viewed from line II-II in Fig. 1.

According to an embodiment, the fermenter is filled with organic material 170 up to a rated level 172. In accordance with an embodiment, a retaining element 174 is mounted in the fermenter 102, the retaining element reducing a movement of the organic material 170 in the fermenter in a surface region 176 of the organic material. According to an embodiment, the retaining element is located at a third location 178. According to an embodiment, the retaining element 174 extends beyond the rated level 172 in a downward direction. For example, in an embodiment, the retaining element 174 has a lower edge which is located below the rated level 172.

In accordance with an embodiment, the third location 178 is located in the first zone 106. In accordance with a third embodiment, the third location is in the second zone 108 (not shown in Fig. 2). The retaining element blocks or at least reduces the movement of organic material 170 in a surface region 176 of the organic material, thereby preventing or at least mitigating that the organic material in the surface region 176 moves faster than the remaining lower portions 180 of the organic material in the fermenter 102.

In accordance with an embodiment, the intermediate outlet of the fermenter 102 is located at the bottom portion of the fermenter 102. In accordance with an embodiment, the bottom portion of the fermenter 102 is made of metal, e.g. steel. Such a bottom formed of metal provides the advantage that the intermediate outlet can easily be realized. In particular, if a free space is provided between the bottom of the fermenter and a fermenter wall, the intermediate outlet 112 can be located in the free space, thereby being easily accessible during installation of the transfer path and, later on, for maintenance of the transfer path and their associated installations.

Fig. 3 shows a lower part of the fermenter of Fig. 2 in cross-sectional view along line III-III of Fig. 2.

In accordance with an embodiment, the fermenter 102 comprises two opposing walls 181, 182 and a preshaped bottom element 184 extending there between. In accordance with an embodiment, the preshaped bottom element 184 is made of metal. In accordance with a further embodiment, the intermediate outlet 112 is formed in the preshaped bottom element 184. In accordance with a further embodiment, a free space is provided between the preshaped bottom element 184 and at least one of the walls 181, 182. The free space 186 facilitates the installation of the transfer path 116.

It should be noted that any entity disclosed herein (e.g. components, units and devices) are not limited to a dedicated entity as described in some embodiments. Rather, the herein disclosed subject matter may be implemented in various ways and with various granularity on device level or software module level while still providing the desired functionality. Further, it should be noted that according to embodiments a separate entity (e.g. a software module, a hardware module or a hybrid module) may be provided for each of the functions disclosed herein. According to other embodiments, an entity (e.g. a software module, a hardware module or a hybrid module (combined software/hardware module)) is configured for providing two or more functions as disclosed herein. For example, a conveyor device as disclosed herein may include a single conveyor entity or two or more conveyor entities, e.g. screw conveyors.

According to an embodiment, the controller unit 118 comprises a processor device including at least one processors for carrying out at least one computer program corresponding to a respective software module.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

In order to recapitulate the above described embodiments of the present invention one can state:

In accordance with embodiments there is provided a method of operating a fermentation device 100 comprising a fermenter 102, the method comprising: moving an organic material through a first zone 106 of the fermenter 102 in an downstream direction 104 while allowing fermentation of the organic material; removing a first part C of the organic material from the first zone 106 at a first location 113; moving a remaining second part F2 of the organic material in the downstream direction 104 past the first location 113 into a second zone 108 of the fermenter 102; re-introducing the first part C of the organic material into the fermenter 102 at a second location 115 upstream the first location 113.

### List of reference signs

- 100: fermentation device
- 102: fermenter
- 104: downstream direction
- 106: first zone
- 108: second zone
- 110: fermenter wall
- 111: length of a cavity of 102
- 112: intermediate outlet
- 113: first location
- 114: inlet
- 115: second location
- 116: transfer path
- 118: mixing unit
- 120: conveyor device
- 122: duct extending from 112
- 124: feeding path
- 125: further inlet
- 126: output duct
- 127: further feeding path
- 132: discharge pump
- 133: fermenter outlet
- 134: dewatering device
- 135: fermenter outlet duct
- 136: press water line
- 138: fresh water supply line
- 140: stirring device
- 142: rotatable shaft
- 144: stirring paddles
- 146: motor
- 148: control unit
- 150: level signal
- 151: level sensor
- 152: temperature signal
- 154: temperature sensor
- 155: control signal to 156
- 156: valve for press water
- 157: control signal to 158
- 158: valve for fresh water
- 159: control signal to 160
- 160: conveyor device for fresh material
- 162: control signal to 120
- 164: control signal to 140
- 170: organic material
- 172: rated level
- 174: retaining element
- 176: surface region
- 178: third location
- 180: lower portion of organic material in 102
- 181, 182: wall
- 184: preshaped bottom element
- 186: free space
- F1:: material flow rate in 106
- F2:: material flow rate in 108
- C:: material flow rate of circulated material
- I:: material flow rate of added fresh material
- L:: material flow rate of added liquid

## Claims

1. Method of operating a fermentation device (100) comprising a fermenter (102), the method comprising:
- moving an organic material (170) through a first zone (106) of the fermenter (102) in an downstream direction while allowing fermentation of the organic material (170);
- removing a first part (C) of the organic material (170) from the first zone (106) at a first location (113);
- moving a remaining second part (F2) of the organic material (170) in the downstream direction past the first location (113) into a second zone (108) of the fermenter (102);
- re-introducing the first part (C) of the organic material into the fermenter (102) at a second location (115) upstream the first location (113).

2. Method according to claim 1, wherein the moving speed of the organic material in the first zone (106) is higher than the moving speed of the organic material in the second zone (107).

3. Method according to any one of claims 1 or 2, further comprising
- adding a further material (L) to the first part (C) of organic material before introduction of the first part (C) of organic material into the fermenter (102); and
- introducing both materials (L, C) together into the fermenter at the second location (115).

4. Method according to claim 3, further comprising, before introducing both materials (L, C) into the fermenter (102), adjusting a dry substance content of a mixture of both materials.

5. Method according to any one of the preceding claims, further comprising
- reducing movement of the organic material (170) in a surface region (176) of the organic material at a third location (178).

6. Fermentation device (100) comprising:
- a fermenter (102) for fermentation of an organic material (170) during a movement of the organic material (170) through the fermenter (102) in a downstream direction (104), the fermenter (102) comprising:
o a first zone (106) with an intermediate outlet (112) for removal of a first part (C) of the organic material (170) from the fermenter (102);
o a second zone (108) for receiving a remaining second part (F2) of the organic material;
o an inlet (114) upstream the intermediate outlet (112);
- the fermentation device (100) further comprising a transfer path (116) between the intermediate outlet (112) and the inlet (114), the transfer path (116) being configured for re-introducing the first part (C) of the organic material into the fermenter (102) through the inlet (114).

7. Fermentation device according to claim 6, the fermenter being configured such that the moving speed of the organic material in the first zone (106) is higher than the moving speed of the organic material in the second zone (108).

8. Fermentation device according to any one of claims 6 or 7, further comprising
- a feeding path (124) running into the transfer path (116) for adding a further material (L) to the first part of organic material (C).

9. Fermentation device according to claim 8, the transfer path (116) further comprising mixing unit (118) for mixing the further material (L) and the first part of organic material (C), thereby providing a mixture of both materials.

10. Fermentation device according to claim 9, comprising a liquid inlet for adding a liquid (L) to thereby adjust a dry substance content of the mixture of both materials.

11. Fermentation device according to claim 10, wherein the liquid (L) comprises or consists of press water and/or fresh water.

12. Fermentation device according to any one of claims 6 to 11, further comprising a conveyor device (120) for conveying the first part of organic material (C) to the inlet (114).

13. Fermentation device according to any one of claims 6 to 12, further comprising
- a retaining element (174) reducing movement of the organic material (170)in the fermenter in a surface region (176) of the organic material at a third location (178).

14. Control unit (148) for a fermentation device, the control unit being configured for carrying out the method according to any one of claims 1 to 5.

15. Computer program being adapted for, when being executed by a data processor device, controlling the method as set forth in any one of the claims 1 to 5.
